# EUROPÄISCHE PATENTANMELDUNG

(11) **EP 2 039 702 A2**
(43) Veröffentlichungstag der Anmeldung: **25.03.2009**
(21) Anmeldenummer: 08161982.7
(22) Anmeldetag: 27.11.2006
(51) Int. Cl.: C07J 41/00, A61K 31/565, A61P 5/28, A61P 5/32, A61P 15/00, A61P 19/10

(54) **Prodrugs erbeta-selektiver Substanzen, Verfahren zu deren Herstellung und diese Verbindungen enthaltende pharmazeutische Zusammensetzungen**

(30) Priorität: 29.11.2005 DE 102005057224
(62) Teilanmeldung aus: 06829357.0
(71) Anmelder: Bayer Schering Pharma Aktiengesellschaft, 13353 Berlin (DE)
(72) Erfinder: Mueller, Gerd, 07745, Jena (DE); Kosemund, Dirk, 07749, Jena (DE); Wyrwa, Ralf, 07751, Rothenstein (DE); Zollner, Thomas, 12161, Berlin (DE); Havlickova, Blanka, 13355, Berlin (DE)

(57) **Zusammenfassung**

Die vorliegende Erfindung stellt Prodrugs von 9α-substituierten Estratrienen der allgemeinen Formel (I) bereit, in denen die Gruppe Z an das Steroid gebunden ist, 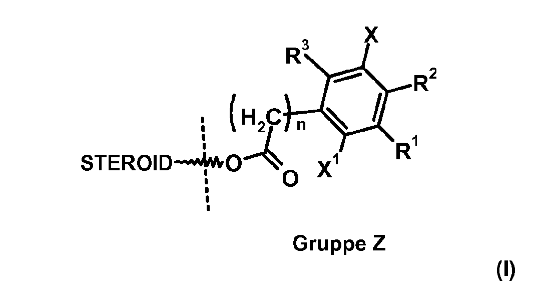 Verfahren zu deren Herstellung, pharmazeutische Zusammensetzungen, die diese Verbindungen enthalten sowie Verwendung derselben.

Die erfindungsgemäßen Verbindungen der allgemeinen Formel I binden nicht an den Estrogenrezeptor α und/ oder β. Sie binden an Carboanhydrasen und hemmen diese Enzyme.

## Beschreibung

Die Erfindung betrifft Prodrugs ERβ-selektiver Substanzen der allgemeinen Formel (I), 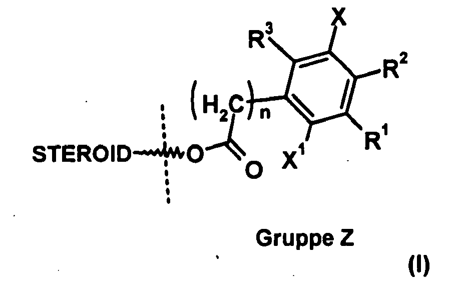 ein Verfahren zu deren Herstellung, diese Verbindungen enthaltende pharmazeutische Zusammensetzungen und deren Verwendung zur Herstellung von Arzneimitteln.

Estrogene spielen im Organismus bei beiden Geschlechtern eine wichtige Rolle. Estrogene sind im reifenden Organismus in die Prägung von Geschlechtsmerkmalen involviert. Estrogene steuern bei beiden Geschlechtern die Umstellungen des Organismus während der Geschlechtsreifung, wie den Wachstumsschub und anschließend die Beendigung des Knochenwachstums. In allen Phasen des Lebens spielen Estrogene bei beiden Geschlechtern im Knochenstoffwechsel eine zentrale Rolle (1, 4). Ihr Verlust führt zum Abbau von Knochensubstanz und birgt das Risiko einer erhöhten Brüchigkeit des Knochens.

Bei der Frau dominieren im Organismus die vom Ovar sezernierten Estrogene. In der Schwangerschaft bildet die Plazenta große Estrogenmengen. Beim Mann entstehen Estrogene überwiegend "peripher" durch die Aromatisierung von Testosteron oder der adrenalen Androgene in verschiedenen Erfolgsorganen, wie dem ZNS, dem Knochen oder dem Darmepithel. Diese Anpassung erlaubt die physiologischen Estrogeneffekte beim Mann bei sehr niedrigen Estradiolspiegeln im Blut. Bei Männern und Frauen mit einem genetischen Defekt der Aromatase oder des Estrogenrezeptors ist der Knochen bezüglich Wachstum und Erhaltung massiv gestört (2).

Während für natürliche Estrogene bedingt durch ihre geringe orale Bioverfügbarkeit die orale Applikation (10) problematisch ist, haben konventionelle chemisch modifizierte Estrogene mit verbesserter Bioverfügbarkeit (bsplw. Ethinylestradiol) oft den Nachteil, eine deutlich gesteigerte Estrogenwirkung in der Leber zu bewirken (3, 9, 10). Diese hepatische Estrogenität betrifft eine Reihe von Funktionen, wie Transportproteine, Fettstoffwechsel, Blutdruckregulation und Gerinnungsfaktoren (5, 7, 11, 12, 14). Auch die für die Erhaltung von Muskulatur und Knochen besonders wichtige Sekretion von IGF-I (8) ist durch hepatische Estrogenwirkungen negativ betroffen (12, 13, 6).

In der WO 01/77139 werden neue 8β-substituierte Estratriene beschrieben, wobei der 8β-Substituent ein gerad- oder verzweigtkettiger, gegebenenfalls teilweise oder vollständig halogenierter Alkyl- oder Alkenylrest mit bis zu 5 Kohlenstoffatomen, ein Ethinyl- oder Prop-1-inylrest sein kann, die als pharmazeutische Wirkstoffe eine höhere *in vitro*- Affinität an Estrogenrezeptorpräparationen von Rattenprostata als an Estrogenrezeptorpräparationen von Rattenuterus zeigen und *in vivo* eine präferentielle Wirkung am Knochen im Vergleich zum Uterus und/ oder ausgeprägte Wirkung hinsichtlich der Stimulierung der Expression von 5HT2a-Rezeptor und -transporter aufweisen. Diese Verbindungen können bevorzugt zur Behandlung von Krankheiten verwendet werden, die durch ein Estrogendefizit bedingt sind.

WO 03/104253 beschreibt neue 9α-substituierte Estratriene mit einem gerad- oder verzweigtkettigen, gegebenenfalls teilweise oder vollständig halogenierten Alkenylrest mit bis zu 6 Kohlenstoffatomen, einem Ethinyl- oder Prop-1-inylrest in Position 9-α, die ebenfalls eine höhere *in vitro* Affinität an Estrogenrezeptor-Präparationen von Rattenprostata als an Estrogenrezeptorpräparationen von Rattenuterus zeigen und in vivo vorzugsweise eine präferentielle Wirkung am Ovar im Vergleich zum Uterus aufweisen. Diese Verbindungen können bevorzugt zur Behandlung von Krankheiten verwendet werden, die durch ein Estrogendefizit bedingt sind.

Aus der WO 01/91797 sind steroidale Verbindungen bekannt, die über eine Gruppe - SO₂NR¹R² an Erythrozyten gebunden werden und sich dort anreichern. Das Konzentrationsverhältnis der Verbindungen zwischen Erythrozyten und Plasma beträgt 10-1000:1, bevorzugterweise 30-1000:1, so dass man von einer Depotbildung in den Erythrozyten sprechen kann. Durch die starke Bindung der Verbindungen an die Erythrozyten wird die Metabolisierung während der Leberpassage vermieden. Nachteilhafterweise sind trotz einer verringerten Metabolisierung mit den angegebenen Dosierungen keine therapierelevanten Wirkstoffspiegel gegeben.

Es ist daher Aufgabe der vorliegenden Erfindung, Prodrugs von ERβ-selektiven Verbindungen bereitzustellen, die die Erβ-selektiven Verbindungen oral bioverfügbar machen.

Diese Aufgabe wird durch Sulfamoylverbindungen von 9α-substituierten Estratrienen der allgemeinen Formel (I) gelöst, in denen die Gruppe Z an das freizusetzende Steroid gebunden ist 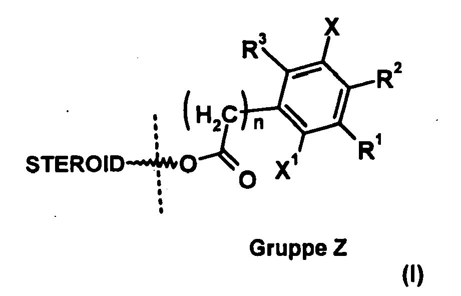 worin n eine Zahl 0 - 4,
- R¹: ein Rest -SO₂NH₂ oder -NHSO₂NH₂,
wobei R², R³ und X, X¹ unabhängig voneinander für ein Wasserstoffatom, ein Halogenatom, eine Nitrilgruppe, eine Nitrogruppe, eine C₁₋₅-Alkylgruppe, eine CₚF₂ₚ₊₁-Gruppe mit p=1-3, eine Gruppe OC(O)-R²⁰, COOR²⁰, OR²⁰, C(O)NHR²⁰ oder OC(O)NH-R²¹ steht,
wobei R²⁰ und R²¹ eine C₁₋₅-Alkylgruppe, eine C₃₋₈-Cycloalkylgruppe, eine Arylgruppe, eine C₁₋₄Alkylenarylgruppe, eine C₁₋₄-Alkylen-C₃₋₈-Cycloalkyl-gruppe oder C₃₋₈-Cycloalkylen-C₁₋₄-Alkylgruppe sind sowie R²⁰ außerdem ein Wasserstoff bedeuten kann, oder
- R²: ein Rest -SO₂NH₂ oder -NHSO₂NH₂,
wobei R¹, R³ und X, X¹ unabhängig voneinander für ein Wasserstoffatom, ein Halogenatom, eine Nitrilgruppe, eine Nitrogruppe, eine C₁₋₅-Alkylgruppe, eine CₚF₂ₚ₊₁-Gruppe mit p=1-3, eine Gruppe OC(O)-R²⁰, COOR²⁰, OR²⁰, C(O)NHR²⁰ oder OC(O)NH-R²¹ steht,
wobei R²⁰ und R²¹ eine C₁₋₅-Alkylgruppe, eine C₃₋₈-Cycloalkylgruppe, eine Arylgruppe, eine C₁₋₄-Alkylenarylgruppe, eine C₁₋₄-Alkylen-C₃₋₈-Cycloalkyl-gruppe oder C₃₋₈-Cycloalkylen-C₁₋₄-Alkylgruppe sind sowie R²⁰ außerdem ein Wasserstoff bedeuten kann, oder
- R³: ein Rest -SO₂NH₂ oder -NHSO₂NH₂,
wobei R¹, R² und X, X¹ unabhängig voneinander für ein Wasserstoffatom, ein Halogenatom, eine Nitrilgruppe, eine Nitrogruppe, eine C₁₋₅-Alkylgruppe, eine CₚF₂ₚ₊₁-Gruppe mit p=1-3, eine Gruppe OC(O)-R²⁰, COOR²⁰, OR²⁰, C(O)NHR²⁰ oder OC(O)NH-R²¹ steht,
wobei R²⁰ und R²¹ eine C₁₋₅-Alkylgruppe, eine C₃₋₈-Cycloalkylgruppe, eine Arylgruppe, eine C₁₋₄-Alkylenarylgruppe, eine C₁₋₄-Alkylen-C₃₋₈-Cycloalkylgruppe oder C₃₋₈-Cycloalkylen-C₁₋₄-Alkylgruppe sind sowie R²⁰ außerdem ein Wasserstoff bedeuten kann, und

STEROID für ein steroidales ABCD-Ringsystem der Formel (A) steht: 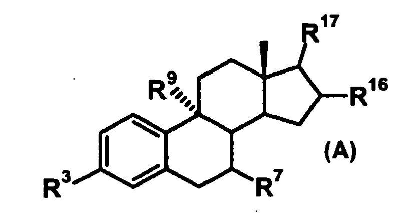 wobei die Reste R⁷, R⁹, R¹⁶ und R¹⁷ folgende Bedeutung besitzen:
- R³: Z und
- R¹⁶: eine OH-Gruppe, eine Tri(C₁-C₄-alkyl)silyloxygruppe oder eine Gruppe OC(O)-R²⁰, oder
- R³: OH, OMe, eine Tri(C₁-C₄-alkyl)silyloxygruppe, eine Gruppe OC(O)-R²⁰ und
- R¹⁶: Z
sowie
- R⁷: ein Wasserstoff- oder Fluoratom, ein Methyl- oder Ethylrest,
- R⁹: ein verzweigter oder geradkettiger, gegebenenfalls teilweise oder vollständig halogenierter Alkyl-, Alkenyl- oder Alkinylrest mit bis zu 3 Kohlenstoffatomen,
- R¹⁷: ein Wasserstoff- oder ein Halogenatom
wobei die Substituenten R⁷, R¹⁶ und R¹⁷ jeweils sowohl in α- als auch in β-Stellung stehen können,
und ihre pharmazeutisch annehmbaren Salze.

Weiterhin umfasst die vorliegende Erfindung die neuen Verbindungen als pharmazeutische Wirkstoffe, deren Herstellung, ihre therapeutische Anwendung und pharmazeutische Darreichungsformen, die die neuen Substanzen enthalten.
Die Erfindung betrifft Estrogenderivate, die selbst nicht an den Estrogenrezeptor binden können und aus denen im Körper das enthaltene Mutterestrogen freigesetzt wird, Verfahren zu deren Herstellung und diese Verbindungen enthaltende pharmazeutische Zusammensetzungen. Die erfindungsgemäßen Verbindungen sind Prodrugs, die nach Verseifung der Estergruppe Z ein ERβ-selektives Estrogen freisetzen (Mutterestrogen).

Durch absolut und relativ stark abgeschwächte Wirkungen über den ER α werden unerwünschte Estrogeneffekte jeder klassischen Estrogentherapie auf den Uterus, die Brustdrüse und die Leber vermieden, wie sie für nicht dissoziierte Estrogene typisch sind. Die erfindungsgemäßen Verbindungen besitzen therapeutisch günstige estrogene Aktivitäten, soweit sie über den ER β vermittelt sind, insbesondere im zentralen Nervensystem, im Kreislaufsystem und im Knochen.

Die erfindungsgemäßen Substanzen werden vorzugsweise zur oralen Therapie eingesetzt. Die erfindungsgemäßen Verbindungen haben gegenüber ihren Mutterestrogenen eine deutlich erhöhte orale Bioverfügbarkeit, eine gesteigerte systemische, aber in der Regel reduzierte hepatische Estrogenität. Durch diese Dissoziation von erwünschten und unerwünschten hormonalen Effekten werden gleichzeitig therapeutisch wirksamere und im Vergleich zum Stand der Technik besser verträgliche Arzneimittel ermöglicht.
Die erfindungsgemäßen Substanzen werden im Körper enzymatisch bzw. hydrolytisch gespalten, wobei keine Steroidsulfatasen (STS), wie zum Beispiel zur Spaltung von Estradiol-3-sulfamat benötigt werden. Damit kann auch die für Estrogen-3-sulfamate typische und für das Erreichen starker estrogener Effekte nachteilige Hemmung der Steroidsulfatase vermieden werden, die für Estrogensulfamate beim Menschen typisch ist. Bei oraler Therapie mit natürlichen Estrogenen (Estradiol, Estradiolvalerat, Estronsulfat, konjugierte Estrogene), aber auch bei der mit Estradiolsulfamat dominieren im Blut hohe Spiegel an Estron (10). Anders als im Zyklus sind die Konzentrationen von Estradiol im Blut niedriger als die von Estron. Dies ist deshalb nachteilig, weil Estron ein schwächer wirksames Estrogen als Estradiol ist.

Ein Vorteil der erfindungsgemäßen Substanzen im Vergleich zu denen im Stand der Technik ist die vorzugsweise Freisetzung des jeweiligen Mutterestrogens, also statt der inaktiven Estronderivate beispielsweise 9α-Ethylestra-3,16α-diol, 9α-Methylestra-3,16α-diol, 9α-Vinylestra-3,16α-diol und 9α-Difluorvinylestra-3,16α-diol als auch deren 17β-fluorierten Analoga.

Die erfindungsgemäßen Verbindungen der allgemeinen Formel (I) oder deren pharmazeutisch annehmbare Salze können als Einzelkomponente in pharmazeutischen Zubereitungen oder in Kombination insbesondere mit Antiestrogenen oder Gestagenen eingesetzt werden. Besonders bevorzugt ist die Kombination mit ERα-selektiven Antiestrogenen oder mit Antiestrogenen, die peripherselektiv wirksam sind, d.h. die die Bluthirnschranke nicht passieren.

Ein therapeutisches Produkt, enthaltend ein Estrogen und ein reines Antiestrogen für die gleichzeitige, sequentielle oder getrennte Anwendung für die selektive Estrogentherapie peri- oder postmenopausaler Zustände ist bereits in der EP-A 0 346 014 beschrieben.

Die Substanzen und die sie enthaltenden Pharmaka sind besonders geeignet für die Behandlung peri- und postmenopausaler Beschwerden, insbesondere Hitzewallungen, Schlafstörungen, Reizbarkeit, Stimmungsschwankungen, Inkontinenz, Vaginalatrophie, hormondefizienzbedingte Gemütserkrankungen. Ebenso sind die Substanzen für die Hormonsubstitution und die Therapie von hormondefizienzbedingten Beschwerden bei chirurgisch, medikamentös oder anders bedingter ovarieller Dysfunktion geeignet. Hierzu gehört auch die Vorbeugung gegen den Knochenmasseverlust bei postmenopausalen Frauen und andropausalen Männern, bei hysterektomierten Frauen oder bei Frauen, die mit LHRH-Antagonisten oder - Agonisten behandelt wurden.

Die erfindungsgemäßen Prodrugs der ERβ-selektiven Agonisten können allein oder in Kombination mit Antiestrogenen, Aromatasehemmern oder Selektiven Estrogen Rezeptor Modulatoren (SERM) für die Behandlung der Prostatahyperplasie verwendet werden, um eine Estrogendeprivation zu vermeiden bzw. um deren Effekte zu reduzieren.

Als Antiestrogen wird bevorzugt 7α-[9-[(4,4,5,5,5-Pentafluoropentyl)sulfinyl]nonyl]estra-1,3,5(10)-trien-3,17β-diol (Fulvestrant) verwendet.

Als zu verwendende Aromatasehemmer kommen folgende in Betracht: Anastrozol, Atamestan, Fadrozol, Formestan, Letrozol.

Als SERM kommen Verbindungen ausgewählt aus der folgenden Gruppe in Betracht: Raloxifen, Tamoxifen, 5-(4-{5-[(RS)-(4,4,5,5,5-Pentafluoropentyl)sulfinyl]pentyl}phenyl)-6-phenyl-8,9-dihydro-7H-benzocyclohepten-2-ol (WO 00/03979).

Die Verbindungen sind auch zur Linderung der Symptome der Andropause und Menopause, d.h. zur männlichen und weiblichen Hormonersatztherapie (HRT) und zwar sowohl zur Prophylaxe als auch zur Behandlung, weiterhin zur Behandlung der mit einer Dysmenorrhoe einhergehenden Beschwerden sowie zur Behandlung der Akne geeignet.

Die Substanzen sind außerdem zur Prophylaxe gegen hormondefizienzbedingten Knochenmasseverlust und Osteoporose, zur Vorbeugung gegen Herzkreislauferkrankungen, insbesondere Gefäßerkrankungen wie Atherosklerose, zur Hemmung der Proliferation der arteriellen Glattmuskelzellen, zur Behandlung des primären pulmonaren Bluthochdrucks einsetzbar.

Weiterhin sind die Substanzen zur Behandlung von entzündlichen und Erkrankungen des Immunsystems insbesondere Autoimmunerkrankungen wie z. B. Rheumatoide Arthritis, Multiple Sklerose, Morbus Crohn oder Endometriose einsetzbar.

Die Verbindungen können insbesondere nach Therapien, die zur Estrogendeprivation führen, beispielsweise nach Behandlung mit Aromatasehemmern oder GnRH-Antagonisten oder -Agonisten, zur Behandlung von arthritischen Symptomen verwendet werden.

Außerdem können die Verbindungen zur Behandlung männlicher Fertilitätsstörungen und prostatischer Erkrankungen Verwendung finden. Die erfindungsgemäßen Verbindungen sind für eine Estrogenbehandlung von Prostatakarzinom geeignet.

Die Verbindungen können auch in Kombination mit dem natürlichen Vitamin D3 oder mit Calcitriol-Analoga für den Knochenaufbau oder als unterstützende Therapie zu Therapien, welche einen Knochenmasseverlust verursachen (beispielsweise eine Therapie mit Glucocorticoiden, Aromatasehemmern, GnRH-Agonisten oder -Antagonisten, Chemotherapie) eingesetzt werden.

Schließlich können die Verbindungen der allgemeinen Formel (I) in Verbindung mit Progesteronrezeptor-Modulatoren, beispielsweise Mesoprogestinen wie Asoprisnil verwendet werden, und zwar insbesondere zur Verwendung in der Hormonersatztherapie und zur Behandlung gynäkologischer Störungen.

Die erfindungsgemäßen Verbindungen gemäß allgemeiner Formel (I) können außerdem verwendet werden für die Behandlung von Alopezie, verursacht beispielsweise durch Chemotherapie.

Unter "C₁₋₅-Alkylgruppe" wird im Sinne der vorliegenden Erfindung ein verzweigter oder geradkettiger Alkylrest mit bis zu 5 Kohlenstoffatomen verstanden, der zum Beispiel durch Halogene wie Fluor, Chlor oder Brom, OH, CN substituiert sein kann. Als Beispiele seien Methyl, Ethyl, n-Propyl, i-Propyl, n-Butyl, i-Butyl, tert.-Butyl oder n-Pentyl genannt.

Die vorstehend genannte "C₃₋₈-Cycloalkylgruppe" bedeutet erfindungsgemäß eine mono- oder bicyclische Gruppe, die zum Beispiel durch Halogene wie Fluor, Chlor oder Brom, OH, CN substituiert sein kann, wie beispielsweise eine Cyclopropyl-, Cyclobutyl-, Cyclopentyl-, Cyclohexyl oder eine Hydroxycyclohexylgruppe.

Unter dem Begriff "Arylgruppe" wird im Sinne der vorliegenden Anmeldung ein substituierter oder unsubstituierter Arylrest mit 6 bis 15 Kohlenstoffatomen, beispielsweise eine Phenylgruppe, eine substituierte Phenylgruppe, wie eine Halogenphenylgruppe oder eine Nitrophenylgruppe, oder eine Naphtylgruppe verstanden.

Unter dem Begriff "C₁₋₄-Alkylenarylgruppe" wird im Sinne der vorliegenden Anmeldung ein disubstituierter Alkylrest verstanden, der mindestens mit einem Arylrest substituiert ist. Beide Reste weisen zusammen 7 bis 15 Kohlenstoffatomen auf, wobei der Arylrest weitere Substituenten, wie beispielsweise ein Halogenatom tragen kann. Beispiele sind eine Benzylgruppe oder eine Halogenbenzylgruppe.

Unter dem Begriff "C₁₋₄-Alkylen-C₃₋₈-Cycloalkylgruppe" wird im Sinne der vorliegenden Anmeldung ein disubstituierter Alkylrest verstanden, der mindestens mit einem C₃₋₈-Cycloalkylrest substituiert ist. Beide Reste weisen zusammen 4 bis 12 Kohlenstoffatomen auf, wobei der Cycloalkylrest weitere Substituenten, wie beispielsweise ein Halogenatom tragen kann. Beispiele sind eine Cyclopentylethyl-, Cyclohexylmethyl-oder Cyclohexylethylgruppe.

Unter dem Begriff "C₃₋₈-Cycloalkylen-C₁₋₄-Alkylgruppe" wird im Sinne der vorliegenden Anmeldung ein disubstituierter C₃₋₈-Cycloalkylenrest verstanden, der mindestens mit einem C₁₋₄-Alkylrest substituiert ist. Beide Reste weisen zusammen 4 bis 12 Kohlenstoffatomen auf, wobei die Gruppe weitere Substituenten, wie beispielsweise ein Halogenatom tragen kann. Beispiele sind eine Propylcyclohexyl- oder Butylcyclohexylgruppe.

Eine Trialkylsilyloxygruppe ist zum Beispiel eine Trimethylsilyloxy- oder tert.-Butyldimethylsilyloxygruppe.

Unter dem Begriff "Halogenatom" wird im Rahmen der vorliegenden Erfindung ein Fluor-, Chlor-, Brom- oder lodatom verstanden. Bevorzugt sind Fluor, Chlor und Brom.

Die Zahl "n" ist vorzugsweise 0,1 oder 2.

R¹ bedeutet vorzugsweise eine Gruppe -SO₂NH₂, wobei R², R³, X¹ und X unabhängig voneinander vorzugsweise ein H-, F-, Cl-Atom, eine OH- oder eine Methoxygruppe sind.

R² bedeutet vorzugsweise eine Gruppe -SO₂NH₂, wobei R¹, R³, X¹ und X unabhängig voneinander vorzugsweise ein H-, F-, Cl-Atom, eine OH- oder eine Methoxygruppe sind.

R³ bedeutet vorzugsweise eine Gruppe -SO₂NH₂, wobei R¹, R², X¹ und X unabhängig voneinander vorzugsweise ein H-, F-, Cl-Atom, eine OH- oder eine Methoxygruppe sind.

X¹ ist vorzugsweise ein H-Atom.

R⁷ ist ein Wasserstoff- oder ein Fluoratom oder ein Methylrest.

R⁹ ist vorzugsweise Methyl, Ethyl, Vinyl, Difluorvinyl, Ethinyl oder Prop-1-inyl.

Besonders bevorzugt sind für R⁹ Ethyl, Vinyl oder Difluorvinyl.

R³ ist vorzugsweise OH, OMe, ein Trimethylsilyloxy-, tert.-Butyldimethylsilyloxy-, ein Benzoat-, ein Sulfamoylbenzoat-, Acetat-, Propionat-, Valerat-, Butcyclat- oder Cyclopentylpropionat-Rest.

R¹⁷ bedeutet vorzugsweise ein Wasserstoff- oder ein Fluoratom.

Besonders bevorzugte Verbindungen im Sinne der Erfindung sind nachfolgend aufgeführt:
1) 3-Hydroxy-9α-vinyl-estra-1,3,5(10)-trien-16α-yl 3'-sulfamoylbenzoat,
2) 3-Hydroxy-17β-fluor-9a-vinyl-estra-1,3,5(10)-trien-16α-yl 3'-sulfamoylbenzoat,
3) 3-Hydroxy-7α-methyl-9α-vinyl-estra-1,3,5(10)-trien-16α-yl 3'-sulfamoylbenzoat,
4) 3-Hydroxy-7α-fluor-9α-vinyl-estra-1,3,5(10)-trien-16α-yl 3'-sulfamoylbenzoat,
5) 3-Acetoxy-9α-vinyl-estra-1,3,5(10)-trien-16α-yl 3'-sulfamoylbenzoat,
6) 3-Acetoxy-17β-fluor-9α-vinyl-estra-1,3,5(10)-trien-16α-yl 3'-sulfamoylbenzoat,
7) 3-Acetoxy-7α-methyl-9α-vinyl-estra-1,3,5(10)-trien-16α-yl 3'-sulfamoylbenzoat,
8) 3-Acetoxy-7α-fluor-9α-vinyl-estra-1,3,5(10)-trien-16α-yl 3'-sulfamoylbenzoat,
9) 3-Hydroxy-9α-vinyl-estra-1,3,5(10)-trien-16α-yl 2'-chlor-5'-sulfamoylbenzoat,
10) 3-Hydroxy-17β-fluor-9α-vinyl-estra-1,3,5(10)-trien-16α-yl 2'-chlor-5'-sulfamoylbenzoat,
11) 3-Hydroxy-7α-methyl-9α-vinyl-estra-1,3,5(10)-trien-16α-yl 2'-chlor-5'-sulfamoylbenzoat,
12) 3-Hydroxy-7α-fluor-9α-vinyl-estra-1,3,5(10)-trien-16α-yl 2'-chlor-5'-sulfamoylbenzoat,
13) 3-Acetoxy-9α-vinyl-estra-1,3,5(10)-trien-16α-yl 2'-chlor-5'-sulfamoylbenzoat,
14) 3-Acetoxy-17β-fluor-9α-vinyl-estra-1,3,5(10)-trien-16α-yl 2'-chlor-5'-sulfamoylbenzoat,
15) 3-Acetoxy-7α-methyl-9α-vinyl-estra-1,3,5(10)-trien-16α-yl 2'-chlor-5'-sulfamoylbenzoat
16) 3-Acetoxy-7α-fluor-9α-vinyl-estra-1,3,5(10)-trien-16α-yl 2'-chlor-5'-sulfamoylbenzoat
17) 3-Hydroxy-9α-vinyl-estra-1,3,5(10)-trien-16α-yl 4'-sulfamoylbenzoat,
18) 3-Hydroxy-17β-fluor-9α-vinyl-estra-1,3,5(10)-trien-16α-yl 4'-sulfamoylbenzoat,
19) 3-Hydroxy-7α-methyl-9α-vinyl-estra-1,3,5(10)-trien-16α-yl 4'-sulfamoylbenzoat,
20) 3-Hydroxy-7α-fluor-9α-vinyl-estra-1,3,5(10)-trien-16α-yl 4'-sulfamoylbenzoat,
21) 3-Acetoxy-9α-vinyl-estra-1,3,5(10)-trien-16α-yl 4'-sulfamoylbenzoat,
22) 3-Acetoxy-17β-fluor-9α-vinyl-estra-1,3,5(10)-trien-16α-yl 4'-sulfamoylbenzoat,
23) 3-Acetoxy-7α-methyl-9α-vinyl-estra-1,3,5(10)-trien-16α-yl 4'-sulfamoylbenzoat
24) 3-Acetoxy-7α-fluor-9α-vinyl-estra-1,3,5(10)-trien-16α-yl 4'-sulfamoylbenzoat

### In-Vitro-Versuche

### a) Blut-Plasma-Konzentrationsverhältnis - Testprinzip und Versuchsbeschreibung:

Die SO₂-NH₂-Gruppe der erfindungsgemäßen Substanzen kann durch Bindung an Carboanhydrasen zu einer Anreicherung in Erythrozyten führen.

### Testprinzip:

Frisch gewonnenes, heparinisiertes Blut einer Ratte wird mit einer definierten Menge an Wirkstoff versetzt. Die Wirkstoffkonzentration im daraus gewonnen Plasma wird gegen eine Kalibrationskurve aus gespiktem (mit bekannter Wirkstoffkonzentration) Plasma gemessen. Das Blut-Plasma Ratio wird berechnet aus der gemessenen Konzentration und der theoretischen Konzentration.

Im Gegensatz zu den in der WO 01/91797 publizierten Ergebnissen liegen die Konzentrationsverhältnisse der erfindungsgemäßen Verbindungen zwischen Erythrozyten und Plasma nicht in einem Bereich von 10-1000:1, sondern im Bereich <10:1. Die Verbindung 17β-Fluor-3-hydroxy-9α-vinylestra-1,3,5(10)-trien-16α-yl 3'-sulfamoylbenzoat akkumuliert mit einem Blut/Plasma-Verhältnis von etwa 3,7 in der Ratte und an humanen Erythrozyten mit 1,2.

### b) Carboanhydrase-Inhibierung - Testprinzip und Versuchsbeschreibung:

Photometrische Bestimmung der Hemmung von humaner Carboanhydrase I oder II durch Sulfonamide oder Sulfamate auf Mikrotiterplatten mit Hilfe der enzymatischen Umwandlung von Nitrophenylacetat mit einem Farbumschlag von farblos nach gelb.

**Tabelle 1: IC₅₀ Hemmwerte humaner Carboanhydrase I und II**

| Inhibitor | CAI | | CAII | |
|---|---|---|---|---|
| | IC₅₀ (nM) | IC₅₀ (nM) Literatur | IC₅₀ (nM) | IC₅₀ (nM) Literatur |
| Estradiol-3-sulfamat | 157 ±10.6 | - | 21.6 ±1.5 | - |
| 17β-Fluor-3-hydroxy-9α-vinylestra-1,3,5(10)-trien-16α-yl 3-sulfamoyl-benzoat | 3700 | - | 720 | - |
| 17β-Fluor-3-hydroxy-9 α -vinylestra-1,3,5(10)-trien-16α-yl benzoate | >10000 | - | >10000 | - |
| 3-Hydroxy-9 α -vinylestra-1,3,5(10)-trien-16α-yl 3-sulfamoylbenzoat | 3400 | - | 490 | - |
| Acetazolamid (bekannter CA-Hemmer) | 1200 | 1900 | 60 | 90¹ |

| | | | | |
|---|---|---|---|---|
| ¹Literatur: C. Landolfi, M. Marchetti, G. Ciocci, and C. Milanese, Journal of Pharmacological and Toxicological Methods 38, 169-172 (1997). | | | | |

Trotz des niedrigen Blut-Plasma-Konzentrationsverhältnisses konnte in allen Fällen eine Bindung (Hemmung) an die beiden Isoenzyme Carboanhydrase CA I und CA II in den Erythrozyten gezeigt werden. Von Bedeutung für die Eigenschaften als Estrogen ist die durch Affinität zu den Carboanhydrasen induzierte Bindung an Erythrozyten. Diese Bindung ist wesentlich für eine reduzierte Extraktion der oral applizierten Substanz bei der ersten Leberpassage. Hohe oder geringere Affinität zu den erythrozytären Carboanhydrasen, raschere oder verzögerte Freisetzung aus diesem Depot und nachfolgende Hydrolyse bestimmen die therapeutische Einsetzbarkeit der erfindungsgemäßen Substanzen. Die erfindungsgemäßen Verbindungen eröffnen dadurch die Möglichkeit bei equimolarer Substanzverabreichung höhere kurz dauernde bzw. gleichmäßigere niedrige und länger dauernde Hormonspiegel zu erreichen. Dadurch werden Wirkstärke und Wirkdauer variiert und eine auf den Organismus abgestimmte Therapie ermöglicht.

### In-Vivo-Versuche

### i.v./ p.o.-Pharmakokinetik

Zur Untersuchung pharmakokinetischer Eigenschaften und Parameter wurden Ratten i.v. und p.o. appliziert und zu verschiedenen Zeitpunkten Blutproben zur Bestimmung gewonnen.

### Testprinzip:

Die verschiedenen pharmakokinetischen Parameter können anhand des Zeitprofils, welches die Substanz zeigt, und unter Zuhilfenahme entsprechender pharmakologischer Software ermittelt werden. Die Konzentration der Prüfsubstanz in den Serum- bzw. Plasma-Proben wurde durch HPLC-UV bzw. durch LCMS/MS bestimmt.
Über den Wiederfund der Substanz pro Zeitpunkt lässt sich der Abbau des Wirkstoffes im Organismus darstellen. Die Abbaugeschwindigkeit dient zur Berechnung der einzelnen pharmakokinetischen Parameter.

Untersuchungen der i.v./p.o.- Kinetik an der Ratte zeigten, dass 17β-Fluor-9α-vinylestra-1,3,5(10)-trien-3,16α-diol nach Freisetzung aus dem Prodrug 17β-Fluor-3-hydroxy-9α-vinylestra-1,3,5(10)-trien-16α-yl 3-sulfamoylbenzoat zu 17 % bioverfügbar war. Nach Gabe des "Mutterestrogens" 17β-Fluor-9α-vinylestra-1,3,5(10)-trien-3,16a-diol konnten nur 6 % detektiert werden.

Diese Testergebnisse eröffnen den erfindungsgemäßen Verbindungen der allgemeinen Formel (I) vielfältige Verwendungsmöglichkeiten für die Hormonersatztherapie (HRT) sowie bei hormonell bedingten Erkrankungen bei Mann und Frau.

Gegenstand der vorliegenden Erfindung sind deshalb auch pharmazeutische Zusammensetzungen, die mindestens eine Verbindung der allgemeinen Formel (I), gegebenenfalls zusammen mit pharmazeutisch verträglichen Hilfs- und Trägerstoffen enthalten.

Die erfindungsgemäßen Substanzen haben gegenüber ihren Mutterestrogenen pharmakodynamisch und pharmakokinetisch verbesserte Eigenschaften, die auf einer verringerten hepatischen Extraktion und gleichmäßigeren und länger anhaltenden Blutspiegeln des freigesetzten Estrogens beruhen.

### Dosierung

Zur erfindungsgemäßen Verwendung werden die Erβ-selektiven Verbindungen der allgemeinen Formel (I) oral verabreicht.

Geeignete Dosierungen der erfindungsgemäßen Verbindungen am Menschen für die Behandlung peri- und postmenopausaler Beschwerden, von hormondefizienzbedingten Beschwerden, von gynäkologischen Störungen wie ovarielle Dysfunktion und Endometriose, von männlichen und weiblichen Fertilitätsstörungen, von hormonbedingten Tumorerkrankungen sowie für die Verwendung in der männlichen und weiblichen Hormonersatztherapie betragen je nach Indikation 5 µg bis 2000 mg pro Tag, je nach Alter und Konstitution des Patienten, wobei die notwendige Tagesdosis durch Einmal- oder Mehrfachabgabe appliziert werden kann.

Für gynäkologische Störungen wie ovarielle Dysfunktion und Endometriose kommen dabei Dosierungen zwischen 0,5 und 100 mg, für die Behandlung von männlichen und weiblichen Fertilitätsstörungen 5 µg bis 50 mg, für hormonbedingte Tumorerkrankungen 5 bis 500 mg sowie für die männliche oder weibliche Hormonersatztherapie 5 µg bis 100 mg in Betracht.

Die pharmazeutischen Zusammensetzungen enthalten neben üblichen Träger- und/oder Verdünnungsmitteln mindestens eine Verbindung der allgemeinen Formel I. Die erfindungsgemäßen Substanzen können auch in Kombination mit einem Gestagen, Antigestagen oder Mesoprogestin therapeutisch eingesetzt werden. Bevorzugt werden die erfindungsgemäßen Substanzen einzeln als Wirkstoff in pharmazeutischen Zubereitungen angewandt.

Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder auch Depotformen.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Verbindungen der allgemeinen Formel I können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten.

Die Verbindungen der allgemeinen Formel I enthaltende Kapseln können beispielsweise hergestellt werden, indem man die Verbindung(en) der allgemeinen Formel I mit einem inerten Träger wie Milchzucker oder Sorbit mischt und in Gelatinekapseln einkapselt.

Die nachfolgenden Beispiele erläutern die vorliegende Erfindung, ohne sie einzuschränken.

### Beispiel 1

### 3-Hydroxy-9-vinylestra-1,3,5(10)-trien-16α-yl 3'-sulfamoylbenzoat

### Stufe 1

### 3,16α-Bis-(tert-butyldimethylsilyloxy)-9α-vinylestra-1,3,5(10)-trien

1,0 g (3,35 mmol) 9α-Vinylestra-1,3,5(10)-trien-3,16α-diol und 2,1 g (13,4 mmol) *tert-*Butyldimethylchlorsilan werden in 25 ml Dimethylformamid vorgelegt und unter Rühren bei Raumtemperatur mit 1,8 g (26,8 mmol) Imidazol portionsweise versetzt. Nach ca. 30 Minuten erhält man eine weiße Suspension. Die Reaktionslösung wird in Eiswasser unter kräftigem Rühren eingegossen. Das ausgefallene Produkt wird abgesaugt, mit Wasser gewaschen und getrocknet. Man erhält 1,62 g ( 92 % d.Th.) 3,16α-Bis-(*tert-*butyldimethylsilyloxy)-9α-vinylestra-1,3,5(10)-trien.

### Stufe 2

### 3-(tert-Butyldimethylsilyloxy)-9α-vinylestra-1,3,5(10)-trien-16α-ol

0,8 g (1,52 mmol) 3,16α-Bis-(*tert*-butyldimethylsilyloxy)-9α-vinylestra-1,3,5(10)-trien werden in 40 ml absolutem Ethanol vorgelegt und unter Rühren sowie Feuchtigkeitsausschluss bei Raumtemperatur mit 1,0 ml (8,0 mmol) Bortrifluorid-etherat versetzt. Nach ca. 60 Minuten wird die Reaktion durch Zugabe von Natriumhydrogenkarbonat-Lösung beendet. Man destilliert Ethanol vom Reaktionsgemisch ab und extrahiert die Reaktionsprodukte mit Essigsäureethylester. Das 3-(*tert*-Butyldimethylsilyloxy)-9α-vinylestra-1,3,5(10)-trien-16α-ol wird durch Säulenchromatographie an Kieselgel 60 gereinigt und isoliert. Man erhält 0,59 g ( 94 % d.Th.).

### Stufe 3

### 3-(tert-Butyldimethylsilyloxy)-9α-vinylestra-1,3,5(10)-trien-16α-yl 3'-sulfamoylbenzoat

0,3 g (0,73 mmol) 3-(*tert*-Butyldimethylsilyloxy)-9α-vinylestra-1,3,5(10)-trien-17β-ol wird in 2 ml Pyridin und 2 ml Methylenchlorid gelöst. Zur Reaktionsmischung werden unter Rühren bei -20°C 0,3 ml (2 mmol) 3-Chlorsulfonylbenzoesäurechlorid zugegeben. Anschließend wird auf Raumtemperatur erwärmt und 15 min gerührt. Zur Reaktionslösung gibt man 25 ml konz. Ammoniaklösung und rührt 15 min intensiv. Mit 10%-iger Salzsäure stellt man den pH-Wert auf 5 ein. Die organischen Lösungsmittel werden weitestgehend abdestilliert. Die ausgefallene Substanz wird abgesaugt und mit Wasser gewaschen. Man erhält 420 mg 3-(*tert*-Butyldimethylsilyloxy)-9α-vinylestra-1,3,5(10)-trien-16α-yl 3'-sulfamoylbenzoat als Rohprodukt.

### Stufe 4

### 3-Hydroxy-9α-vinylestra-1,3,5(10)-trien-16α-yl 3'-sulfamoylbenzoat

420 mg Rohprodukt aus Stufe 3 werden in 10 ml Tetrahydrofuran gelöst. Unter Rühren gibt man bei Raumtemperatur 420 mg (1,3 mmol) Tetra-*n*-butylammoniumfluorid-Trihydrat zu. Nach 1 Stunde werden 40 ml Wasser eingerührt, Tetrahydrofuran abdestilliert und das Reaktionsprodukt mit Essigsäureethylester extrahiert. Nach Isolierung des Reaktionsproduktes durch Abdestillieren des Essigsäureethylesters reinigt man das Rohprodukt durch Chromatographie an Kieselgel 60. Man erhält 295 mg 3-Hydroxy-9α-vinylestra-1,3,5(10)-trien-16α-yl 3'-sulfamoylbenzoat (84 % d.Th. bezogen auf 3-(*tert-*Butyldimethylsilyloxy)-9a-vinylestra-1,3,5(10)-trien-17β-ol).
¹H-NMR (400 MHz, DMSO-d₆, TMS):
9,00 (s, 3-OH); 8,39 (s, 1H); 8,16 (m, 1H); 8,08 (m, 1H), 7,73 (m, 1H); 7.55 (s, 2H, NH₂); 7,00 (d, J = 8,6 Hz, H-1); 6,53 (dd, J = 8,6/2,7 Hz, H-2); 6,43 (d, J = 2,7 Hz, H-4); 6,28 (dd, J = 17,2/10,5 Hz, -CH=CH₂); 5,44 (m, 1H, H-16β); 5,02 (dd, J = 10,5/1,9 Hz, -CH=CH₂); 4,48 (dd, J = 17,2/1,9 Hz, -CH=CH₂); 0,82 (s, 3H, H-18),

### Beispiel 2

### 3-Hydroxy-17β-fluor-9α-vinylestra-1,3,5(10)-trien-17β-yl 3'-sulfamoylbenzoat

### Stufe 1

### 3,16α-Bis-(tert-butyldimethylsilyloxy)-17β-fluor-9α-vinylestra-1,3,5(10)-trien

1,0 g (3,16 mmol) 9α-Vinylestra-1,3,5(10)-trien-3,16α-diol und 2,1 g (13,4 mmol) *tert-*Butyldimethylchlorsilan werden in 25 ml Dimethylformamid vorgelegt und unter Rühren bei Raumtemperatur mit 1,8 g (26,8 mmol) Imidazol portionsweise versetzt. Nach ca. 30 Minuten erhält man eine weiße Suspension. Die Reaktionslösung wird in Eiswasser unter kräftigem Rühren eingegossen. Das ausgefallene Produkt wird abgesaugt, mit Wasser gewaschen und getrocknet. Man erhält 1,53 g ( 87 % d.Th.) 3,16α-Bis-(*tert-*butyldimethylsilyloxy)-17β-fluor-9α-vinylestra-1,3,5(10)-trien.

### Stufe 2

### 3-(tert-Butyldimethylsilyloxy)-17β-fluor-9α-vinylestra-1,3,5(10)-trien-16α-ol

1,53 g (2,81 mmol) 3,16α-Bis-(*tert*-butyldimethylsilyloxy)-17β-fluor-9α-vinylestra-1,3,5(10)-trien werden in 40 ml absolutem Ethanol vorgelegt und unter Rühren sowie Feuchtigkeitsausschluss bei Raumtemperatur mit 1,8 ml (14,8 mmol) Bortrifluorid-etherat versetzt. Nach ca. 60 Minuten wird die Reaktion durch Zugabe von Natriumhydrogenkarbonat-Lösung beendet. Man destilliert Ethanol vom Reaktionsgemisch ab und extrahiert die Reaktionsprodukte mit Essigsäureethylester. Durch Säulenchromatographie an Kieselgel 60 wird das 3-(*tert*-Butyldimethylsilyloxy)-17β-fluor-9α-vinylestra-1,3,5(10)-trien-16α-ol gereinigt und durch Abdestillieren des Eluenten isoliert. Man erhält 1,21 g ( 91 % d.Th.).

### Stufe 3

### 3-(tert-Butyldimethylsilyloxy)-17β-fluor-9α-vinylestra-1,3,5(10)-trien-16α-yl 3'-sulfamoyl-benzoat

1,21 g (2,81 mmol) 3-(*tert*-Butyldimethylsilyloxy)-17β-fluor-9α-vinylestra-1,3,5(10)-trien-17β-ol wird in 8 ml Pyridin und 8 ml Methylenchlorid gelöst. Zur Reaktionsmischung werden unter Rühren bei -20°C 1,2 ml (8,0 mmol) 3-Chlorsulfonylbenzoesäurechlorid zugegeben. Anschließend wird auf Raumtemperatur erwärmt und 15 min gerührt. Zur Reaktionslösung gibt man 25 ml konz. Ammoniaklösung und rührt 15 min intensiv. Mit 10%-iger Salzsäure stellt man den pH-Wert auf 5 ein. Die organischen Lösungsmittel werden weitestgehend abdestilliert. Die abgeschiedene Substanz wird nach dem Abtrennen mit Wasser gewaschen. Man erhält 1,62 g 3-(*tert*-Butyldimethylsilyloxy)-17β-fluor-9α-vinylestra-1,3,5(10)-trien-16α-yl 3'-sulfamoylbenzoat als ein öliges Rohprodukt.

### Stufe 4

### 3-Hydroxy-17β-fluor-9α-vinylestra-1,3,5(10)-trien-16α-yl 3'-sulfamoylbenzoat

1,62 g Rohprodukt aus Stufe 3 werden in 30 ml Tetrahydrofuran gelöst. Unter Rühren gibt man bei Raumtemperatur 1,62 g (5,0 mmol) Tetra-*n*-butylammoniumfluorid-Trihydrat zu. Nach 1 Stunde wird 40 ml Wasser eingerührt, Tetrahydrofuran abdestilliert und das Reaktionsprodukt mit Essigsäureethylester extrahiert. Nach Isolierung des Reaktionsproduktes durch Abdestillieren des Essigsäureethylesters reinigt man das Rohprodukt durch Chromatographie an Kieselgel 60. Man erhält 1,14 g 3-Hydroxy-17β-fluor-9α-vinylestra-1,3,5(10)-trien-16α-yl 3'-sulfamoylbenzoat (81 % d.Th. bezogen auf 3-(*tert-*Butyldimethylsilyloxy)-17β-fluor-9α-vinylestra-1,3,5(10)-then-17β-ol).
¹H-NMR (400 MHz, DMSO-d₆, TMS):
9,07 (s, 3-OH); 8,42 (s, 1H); 8,22 (d, J = 7,8 Hz, 1H); 8,09 (d, J = 7,8 Hz, 1H); 7,76 (t, J = 7,8 Hz, 1H); 7.56 (s, 2H, NH₂); 7,00 (d, J = 8,6 Hz, H-1); 6,52 (dd, J = 8,6/2,4 Hz, H-2); 6,42 (d, J = 2,4 Hz, H-4); 6,29 (dd, J = 17,2/10,7 Hz, -CH=CH₂); 5,39 (m, 1H, H-16β); 5,03 (dd, J = 10,7/1,9 Hz, -CH=CH₂); 4,95 (dd, J = 54,3/5,1 Hz, H-17α); 4,45 (dd, J = 17,2/1,9 Hz, - CH=CH₂); 0,92 (d, J = 1.0 Hz, 3H, H-18),

### Referenzen:

1. Cummings SR, Browner WS, Bauer D, Stone K, Ensrud K, Jamal S and Ettinger B (1998), Endogenous hormones and the risk of hip and vertebral fractures among older women. N. Engl. J. Med. 339, 733-38.
2. Frank GR (1995), The role of estrogen in pubertal skeletal physiology: epiphyseal maturation and mineralization of the skeleton. Acta Paediatr. 84(6), 627-30.
3. Goldzieher JW (1990), Selected aspects of the pharmacokinetics and metabolism of ethinyl estrogens and their clinical implications. Am. J. Obstet. Gynecol. 163, 318-22.
4. Gustafsson JA (2000), Novel aspects of estrogen action. J. Soc. Gynecol. Investig. 7, S8-S9.
5. Helmer OM and Griffith RS (1952), The effect of the administration of estrogens on the renin-substrate (hypertensinogen) content on rat plasma. Endocrinology 51, 421-6.
6. Kelly JJ, Rajkovic IA, O'Sullivan AJ, Semia C and Ho KKY (1993), Effects of different oral oestrogen formulations on insulin-like growth factor-I, growth hormone and growth hormone binding protein in post-menopausal women. Clin. Endocrinol. 39, 561-67.
7. Krattenmacher R, Knauthe R, Parczyk K, Walker A, Hilgenfeldt U and Fritzemeier K-H (1994), Estrogen action on hepatic synthesis of angiotensinogen and IGF-I: direct and indirect estrogen effects. J. Steroid. Biochem. Mol. Biol. 48, 207-14.
8. Le Roith and Butler AA (1999), Insulin-like growth factors in pediatric health and disease. J. Clin. Endocrinol. Metab. 84, 4355-61.
9. Mandel FP, Geola FL, Lu JKH, Eggena P, Sambhi MP, Hershman JM and Judd HL (1982), Biologic effects of various doses of ethinyl estradiol in postmenopausal women. Obstet. Gynecol. 59, 673-9.
10. Mashchak CA, Lobo RA, Dozono-Takano R, Eggena P, Nakamura RM, Brenner PF and Mishell DR Jr (1982), Comparison of pharmacodynamic properties of various estrogen formulations. Am. J. Obstet. Gynecol. 144, 511-18.
11.Oelkers WKH (1996), Effects of estrogens and progestagens on the reninaldosterone system and blood pressure. Steroids 61, 166-71.
12.O'Sullivan AJ and Ho KKY (1995), A comparison of the effects of oral and transdermal estrogen replacement on insulin sensitivity in postmenopausal women. J. Clin. Endocrinol. Metab. 80, 1783-8.
13. Span JPT, Pieters GFFM, Sweep CGJ, Hermus ARMM and Smals AGH (2000), Gender difference in insulin-like growth factor I response to growth hormone (GH) treatment in GH-deficient adults: role of sex hormone replacement. J. Clin. Endocrinol. Metab. 85, 1121-5.
14. von Schoultz B, Carlström K, Collste L, Eriksson A, Henriksson P, Pousette A and Stege R (1989), Estrogen therapy and liver function - metabolic effects of oral and parenteral administration. Prostate 14, 389-95.

## Patentansprüche

1. Verwendung von Verbindungen der allgemeinen Formel (I) 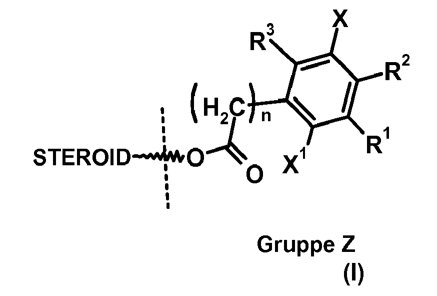 worin n eine Zahl 0 - 4,
R¹ ein Rest -SO₂NH₂ oder -NHSO₂NH₂,
wobei R², R³ und X, X¹ unabhängig voneinander für ein Wasserstoffatom, ein Halogenatom, eine Nitrilgruppe, eine Nitrogruppe, eine C₁₋₅-Alkylgruppe, eine CₚF₂ₚ₊₁-Gruppe mit p=1-3, eine Gruppe OC(O)-R²⁰ , COOR²⁰, OR²⁰, C(O)NHR²⁰ oder OC(O)NH-R²¹ steht,
wobei R²⁰ und R²¹ eine C₁₋₅-Alkylgruppe, eine C₃₋₈-Cycloalkylgruppe, eine Arylgruppe, eine C₁₋₄-Alkylenarylgruppe, eine C₁₋₄-Alkylen-C₃₋₈-Cycloalkylgruppe oder C₃₋₈-Cycloalkylen-C₁₋₄-Alkylgruppe sind sowie R²⁰ außerdem ein Wasserstoff bedeuten kann, oder
R² ein Rest -SO₂NH₂ oder -NHSO₂NH₂,
wobei R¹, R³ und X, X¹ unabhängig voneinander für ein Wasserstoffatom, ein Halogenatom, eine Nitrilgruppe, eine Nitrogruppe, eine C₁₋₅-Alkylgruppe, eine CₚF₂ₚ₊₁-Gruppe mit p=1-3, eine Gruppe OC(O)-R²⁰ , COOR²⁰, OR²⁰, C(O)NHR²⁰ oder OC(O)NH-R²¹ steht,
wobei R²⁰ und R²¹ eine C₁₋₅-Alkylgruppe, eine C₃₋₈-Cycloalkylgruppe, eine Arylgruppe, eine C₁₋₄-Alkylenarylgruppe, eine C₁₋₄-Alkylen-C₃₋₈-Cycloalkylgruppe oder C₃₋₈-Cycloalkylen-C₁₋₄-Alkylgruppe sind sowie R²⁰ außerdem ein Wasserstoff bedeuten kann, oder
R³ ein Rest -SO₂NH₂ oder -NHSO₂NH₂,
wobei R¹, R² und X, X¹ unabhängig voneinander für ein Wasserstoffatom, ein Halogenatom, eine Nitrilgruppe, eine Nitrogruppe, eine C₁₋₅-Alkylgruppe, eine CₚF₂ₚ₊₁-Gruppe mit p=1-3,
eine Gruppe OC(O)-R²⁰ , COOR²⁰, OR²⁰, C(O)NHR²⁰ oder OC(O)NH-R²¹ steht,
wobei R²⁰ und R²¹ eine C₁₋₅-Alkylgruppe, eine C₃₋₈-Cycloalkylgruppe, eine Arylgruppe, eine C₁₋₄-Alkylenarylgruppe, eine C₁₋₄-Alkylen-C₃₋₈-Cycloalkylgruppe oder C₃₋₈-Cycloalkylen-C₁₋₄-Alkylgruppe sind sowie R²⁰ außerdem ein Wasserstoff bedeuten kann, und
STEROID für ein steroidales ABCD-Ringsystem der Formel (A) steht: 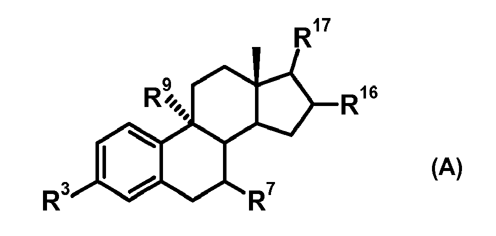 wobei die Reste R⁷, R⁹, R¹⁶ und R¹⁷ folgende Bedeutung besitzen:
R³ Z und
R¹⁶ eine OH-Gruppe, eine Tri(C₁-C₄-alkyl)silyloxygruppe oder eine Gruppe OC(O)-R²⁰, oder
R³ OH, OMe, eine Tri(C₁-C₄-alkyl)silyloxygruppe, eine Gruppe OC(O)-R²⁰ und
R¹⁶ Z
sowie
R⁷ ein Wasserstoff- oder Fluoratom, ein Methyl- oder Ethylrest,
R⁹ ein verzweigter oder geradkettiger, gegebenenfalls teilweise oder vollständig halogenierter Alkyl-, Alkenyl- oder Alkinylrest mit bis zu 3 Kohlenstoffatomen,
R¹⁷ ein Wasserstoff- oder ein Halogenatom
wobei die Substituenten R⁷, R¹⁶ und R¹⁷ jeweils sowohl in □- als auch in □-Stellung stehen können,
und ihrer pharmazeutisch annehmbaren Salze zur Herstellung von Arzneimitteln zur Behandlung von Alopezie.

2. Verwendung von Verbindungen nach Anspruch 1, **dadurch gekennzeichnet, dass** n 0, 1 oder 2 ist.

3. Verwendung von Verbindungen nach Anspruch 1 oder 2, **dadurch gekennzeichnet, dass** jeweils ein Rest R¹, R² oder R³ eine Gruppe -SO₂NH₂ darstellt.

4. Verwendung von Verbindungen nach einem der Ansprüche 1 oder 2, **dadurch gekennzeichnet, dass** R¹ eine Gruppe-SO₂NH₂ oder-NHSO₂NH₂ darstellt.

5. Verwendung von Verbindungen nach Anspruch 4, **dadurch gekennzeichnet, dass** R¹ eine Gruppe -SO₂NH₂ darstellt.

6. Verwendung von Verbindungen nach einem der Ansprüche 1 bis 5, **dadurch gekennzeichnet, dass,** wenn einer der Reste R¹, R², R³ nicht -SO₂NH₂ oder - NHSO₂NH₂ bedeutet, die jeweils anderen beiden Reste von R¹, R², R³ sowie X und X¹ unabhängig voneinander für ein Wasserstoff-, Fluor-, Chloratom, eine Hydroxy- oder eine Methoxygruppe stehen.

7. Verbindungen nach einem der Ansprüche 1 bis 6, **dadurch gekennzeichnet, dass** R⁹ ein Methyl-, Ethyl-, Vinyl- oder Difluorvinylrest ist.

8. Verwendung von
1.) 3-Hydroxy-9α-vinyl-estra-1,3,5(10)-trien-16α-yl 3'-sulfamoylbenzoat,
2.) 3-Hydroxy-17β-fluor-9α-vinyl-estra-1,3,5(10)-trien-16α-yl 3'-sulfamoyl-benzoat,
3.) 3-Hydroxy-7α-methyl-9α-vinyl-estra-1,3,5(10)-trien-16α-yl 3'-sulfamoyl-benzoat,
4.) 3-Hydroxy-7α-fluor-9α-vinyl-estra-1,3,5(10)-trien-16α-yl 3'-sulfamoylbenzoat,
5.) 3-Acetoxy-9α-vinyl-estra-1,3,5(10)-trien-16α-yl 3'-sulfamoylbenzoat,
6.) 3-Acetoxy-17β-fluor-9α-vinyl-estra-1,3,5(10)-trien-16α-yl 3'-sulfamoyl-benzoat,
7.) 3-Acetoxy-7α-methyl-9α-vinyl-estra-1,3,5(10)-trien-16α-yl 3'-sulfamoy-Ibenzoat,
8.) 3-Acetoxy-7α-fluor-9α-vinyl-estra-1,3,5(10)-trien-16α-yl 3'-sulfamoylbenzoat,
9.) 3-Hydroxy-9α-vinyl-estra-1,3,5(10)-trien-16α-yl 2'-chlor-5'-sulfamoylbenzoat,
10.) 3-Hydroxy-17β-fluor-9α-vinyl-estra-1,3,5(10)-trien-16α-yl 2'-chlor-5'-sulfamoylbenzoat,
11.) 3-Hydroxy-7α-methyl-9α-vinyl-estra-1,3,5(10)-trien-16α-yl 2'-chlor-5'-sulfamoylbenzoat,
12.) 3-Hydroxy-7α-fluor-9a-vinyl-estra-1,3,5(10)-trien-16α-yl 2'-chlor-5'-sulfamoylbenzoat,
13.) 3-Acetoxy-9α-vinyl-estra-1,3,5(10)-trien-16α-yl 2'-chlor-5'-sulfamoylbenzoat,
14.) 3-Acetoxy-17β-fluor-9α-vinyl-estra-1,3,5(10)-trien-16α-yl 2'-chlor-5'-sulfamoylbenzoat,
15.) 3-Acetoxy-7α-methyl-9α-vinyl-estra-1,3,5(10)-trien-16α-yl 2'-chlor-5'-sulfamoylbenzoat
16.) 3-Acetoxy-7α-fluor-9α-vinyl-estra-1,3,5(10)-trien-16α-yl 2'-chlor-5'-sulfamoyl-benzoat
17.) 3-Hydroxy-9α-vinyl-estra-1,3,5(10)-trien-16α-yl 4'-sulfamoylbenzoat,
18.) 3-Hydroxy-17β-fluor-9α-vinyl-estra-1,3,5(10)-trien-16α-yl 4'-sulfamoyl-benzoat,
19.) 3-Hydroxy-7α-methyl-9α-vinyl-estra-1,3,5(10)-trien-16α-yl 4'-sulfamoyl-benzoat,
20.) 3-Hydroxy-7α-fluor-9α-vinyl-estra-1,3,5(10)-trien-16α-yl 4'-sulfamoylbenzoat,
21.) 3-Acetoxy-9α-vinyl-estra-1,3,5(10)-trien-16α-yl 4'-sulfamoylbenzoat,
22.) 3-Acetoxy-17β-fluor-9a-vinyl-estra-1,3,5(10)-trien-16α-yl 4'-sulfamoyl-benzoat,
23.) 3-Acetoxy-7α-methyl-9α-vinyl-estra-1,3,5(10)-trien-16α-yl 4'-sulfamoyl-benzoat
24.) 3-Acetoxy-7α-fluor-9α-vinyl-estra-1,3,5(10)-trien-16α-yl 4'-sulfamoylbenzoat
25.) 9α-Ethylestra-3,16α-diol
26.) 9α-Methylestra-3,16α-diol
27.) 9α-Vinylestra-3,16α-diol
28.) 9α-Difluorvinylestra-3,16α-diol
29.) 9α-Ethyl,17β-fluorestra-3,16α-diol
30.) 17β-Fluor,9α-methylestra-3,16α-diol
31.) 17β-Fluor,9α-vinylestra-3,16α-diol
32.) 9α-Difluorvinyl,17β-fluor, estra-3,16α-diol
gemäß Anspruch 1.
